# EUROPEAN PATENT SPECIFICATION

(11) **EP 2 043 553 B1**
(45) Date of publication and mention of the grant of the patent: **24.03.2010**
(21) Application number: 07796718.0
(22) Date of filing: 06.07.2007
(51) Int. Cl.: A61F 2/06

(54) **STENT CRACK REDUCTION**
STENT-RISS-REDUKTION
REDUCTION DE FISSURES SUR UN STENT

(30) Priority: 10.07.2006 US 819827 P; 29.06.2007 US 824155
(43) Date of publication of application: 08.04.2009
(73) Proprietor: Abbott Cardiovascular Systems Inc., Santa Clara, CA 95054-2807 (US)
(72) Inventor: GALE, David C., San Jose, California 95117 (US); CASTRO, Daniel A., Santa Clara, California 95051 (US); HUANG, Bin, Pleasanton, California 94566 (US)
(74) Representative: ZBM Patents
(86) International application number: PCT/US2007/015558
(87) International publication number: WO 2008/008283

(56) References cited:
- US-A- 5 147 385
- US-A- 5 292 321
- US-A- 5 670 161
- US-B1- 6 607 553
- US-B1- 7 008 446

## Description

### BACKGROUND OF THE INVENTION

### Field of the Invention

This invention relates to implantable medical devices, such as stents.

### Description of the State of the Art

This invention relates to radially expandable endoprostheses, which are adapted to be implanted in a bodily lumen. An "endoprosthesis" corresponds to an artificial device that is placed inside the body. A "lumen" refers to a cavity of a tubular organ such as a blood vessel.

A stent is an example of such an endoprosthesis. Stents are generally cylindrically shaped devices, which function to hold open and sometimes expand a segment of a blood vessel or other anatomical lumen such as urinary tracts and bile ducts. Stents are often used in the treatment of atherosclerotic stenosis in blood vessels. "Stenosis" refers to a narrowing or constriction of the diameter of a bodily passage or orifice. In such treatments, stents reinforce body vessels and prevent restenosis following angioplasty in the vascular system. "Restenosis" refers to the reoccurrence of stenosis in a blood vessel or heart valve after it has been treated (as by balloon angioplasty, stenting, or valvuloplasty) with apparent success.

The treatment of a diseased site or lesion with a stent involves both delivery and deployment of the stent. "Delivery" refers to introducing and transporting the stent through a bodily lumen to a region, such as a lesion, in a vessel that requires treatment. "Deployment" corresponds to the expanding of the stent within the lumen at the treatment region. Delivery and deployment of a stent are accomplished by positioning the stent about one end of a catheter, inserting the end of the catheter through the skin into a bodily lumen, advancing the catheter in the bodily lumen to a desired treatment location, expanding the stent at the treatment location, and removing the catheter from the lumen.

In the case of a balloon expandable stent, the stent is mounted about a balloon disposed on the catheter. Mounting the stent typically involves compressing or crimping the stent onto the balloon. The stent is then expanded by inflating the balloon, for example, by pumping a fluid into the catheter and balloon. To inflate or deflate the balloon, a physician typically uses an inflation device, such as a syringe, placed in fluid communication with the interior of the balloon. The physician uses one hand to grasp the syringe body and the other hand to maneuver the plunger to pressurize or depressurize the inflation fluid as required. Manually operated syringe-type inflation systems of the type described are manufactured and sold by Abbott Vascular of Santa Clara, Calif. under the trademark INDEFLATOR. After deployment, the balloon may then be deflated and the catheter withdrawn. In the case of a self-expanding stent, the stent may be secured to the catheter via a constraining member such as a retractable sheath or a sock. When the stent is in a desired bodily location, the sheath may be withdrawn which allows the stent to self expand. The expansion of self-expandable stents may also be balloon-assisted. In this case, a balloon is inflated to partially expand the stent, but not enough to fully deploy the stent. The stent then fully expands and deploys without further assistance from a balloon.

Additionally, accurate stent placement is facilitated by real time visualization of the delivery of a stent. A cardiologist or interventional radiologist can track the delivery catheter through the patient's vasculature and precisely place the stent at the site of a lesion. This is typically accomplished by fluoroscopy or similar x-ray visualization procedures. For a stent, catheter, or balloon to be fluoroscopically visible they must be more absorptive of x-rays than the surrounding tissue. Alternatively, a fluid visible to magnetic resonance imaging (MRI) can be used. One way of accomplishing this is to use a fluid that is fluoroscopically visible to inflate a balloon. Such fluids are referred to as contrast agents.

The stent must be able to satisfy a number of mechanical requirements. First, the stent must be capable of withstanding the structural loads, namely radial compressive forces, imposed on the stent as it supports the walls of a vessel. Therefore, a stent must possess adequate radial strength. Radial strength, which is the ability of a stent to resist radial compressive forces, is due to strength and rigidity around a circumferential direction of the stent. Radial strength and rigidity, therefore, may also be described as, hoop or circumferential strength and rigidity.

Once expanded, the stent must adequately maintain its size and shape throughout its service life despite the various forces that may come to bear on it, including the cyclic loading induced by the beating heart. For example, a radially directed force may tend to cause a stent to recoil inward. Generally, it is desirable to minimize recoil. In addition, the stent must possess sufficient flexibility to allow for crimping, expansion, and cyclic loading. Longitudinal flexibility is important to allow the stent to be maneuvered through a tortuous vascular path and to enable it to conform to a deployment site that may not be linear or may be subject to flexure. Finally, the stent must be biocompatible so as not to trigger any adverse vascular responses.

The structure of a stent is typically composed of scaffolding that includes a pattern or network of interconnecting structural elements often referred to in the art as struts or bar arms. The scaffolding can be formed from wires, tubes, or sheets of material rolled into a cylindrical shape. The scaffolding is designed so that the stent can be radially compressed (to allow crimping) and radially expanded (to allow deployment). A conventional stent is allowed to expand and contract through movement of individual structural elements of a pattern with respect to each other.

Additionally, a medicated stent may be fabricated by coating the surface of either a metallic or polymeric scaffolding with a polymeric carrier that includes an active or bioactive agent or drug. Polymeric scaffolding may also serve as a carrier of an active agent or drug.

Furthermore, it may be desirable for a stent to be biodegradable. In many treatment applications, the presence of a stent in a body may be necessary for a limited period of time until its intended function of, for example, maintaining vascular patency and/or drug delivery is accomplished. Therefore, stents fabricated from biodegradable, bioabsorbable, and/or bioerodable materials such as bioabsorbable polymers should be configured to completely erode only after the clinical need for them has ended.

A potential problem with polymeric stents is that their struts or bar arms can crack during crimping and expansion. This is especially the case with brittle polymers. The localized portions of the stent pattern subjected to substantial deformation during crimping and expansion tend to be the most vulnerable to failure.

Another potential problem with polymeric stents is creep. Creep is a consequence of the viscoelastic nature of polymeric materials. Creep refers to the gradual deformation that occurs in a polymeric material subjected to an applied load. Creep occurs even when the applied load is constant. Creep in a polymeric stent reduces the effectiveness of a stent in maintaining a desired vascular patency. In particular, creep contributes to recoil, allowing inward radial forces to permanently deform a stent radially inward.

Therefore, it is desirable for a stent to have flexibility and resistance to cracking during deployment. It is also advantageous for a stent to be rigid and resistant to creep after deployment.

Document US5670161 discloses a stent delivery assembly, comprising a balloon catheter and an optical fiber for introducing electromagnetic radiation through the catheter. The balloon may be coated with a material, such as a dye, that absorbs the electromagnetic radiation so as to heat a polymer stent mounted on the balloon. The stent is heated at the point of deployment until above the glass-transition temperature of the polymer and then expanded by inflating the balloon.

Furthermore, document US6607553 discloses coating a polymer stent, or selected portions thereof, with a radiation-absorbing material, such as indocyanine green or carbon black, so that the stent or said selected portions can be heated above the glass-transition temperature of the polymer before expansion by a balloon.

Document US7008446 discloses a stent delivery balloon catheter with electrical heating elements incorporated in the catheter or the balloon surface for thermally moulding the stent in-situ.

### SUMMARY OF THE INVENTION

Apparatuses, and systems for delivering a stent at an elevated temperature are disclosed herein.

According to some embodiments, an assembly for delivering a stent mounted on an expandable member within a bodily lumen comprises: means for conveying infrared energy to an expandable member coupled to a catheter assembly, allowing the heated expandable member to increase a temperature of a stent mounted on the expandable member, wherein the increase in temperature increases the flexibility of the stent such that formation of cracks in the stent upon its expansion is reduced or eliminated.

In some embodiments the stent is capable of absorbing infrared energy, thereby heating itself and increasing its flexibility.

### BRIEF DESCRIPTION OF THE DRAWINGS

FIG. 1 depicts a stent.
FIG. 2A-2C depict a heated stent assembly according to some embodiments of the invention.
FIG. 3A-3C depict a heated stent delivery system according to some embodiments of the invention.
FIGs. 4A-4B depict a pressure delivery system according to some embodiments of the invention.

### DETAILED DESCRIPTION OF THE INVENTION

The structural members of polymeric stents can crack during crimping and radial expansion, sometimes leading to mechanical failure of a stent after deployment. Such cracking or rupturing can cause a stent strut to dislodge. The dislodged stent can cause an embolism in the lumen of the tubular organ. In addition, a dislodged stent can orient itself perpendicular to blood flow thereby causing thrombosis.

Rigid polymers are particularly susceptible to cracking when deformed such as when a stent is radially expanded. Polymers below their glass transition temperature tend to be rigid. The "glass transition temperature," Tg, is the temperature at which the amorphous domains of a polymer change from a brittle vitreous state to a solid deformable or ductile state at atmospheric pressure. In other words, the Tg corresponds to the temperature where the onset of segmental motion in the chains of the polymer occurs. When an amorphous or semicrystalline polymer is exposed to an increasing temperature, the coefficient of expansion and the heat capacity of the polymer both increase as the temperature is raised, indicating increased molecular motion. As the temperature is raised the actual molecular volume in the sample remains constant, and so a higher coefficient of expansion points to an increase in free volume associated with the system and therefore increased freedom for the molecules to move. The increasing heat capacity corresponds to an increase in heat dissipation through movement. Tg of a given polymer can be dependent on the heating rate and can be influenced by the thermal history of the polymer. Furthermore, the chemical structure of the polymer heavily influences the glass transition by affecting mobility.

Below the Tg of a polymer, polymer segments may not have sufficient energy to move past one another. A polymer in a rigid state may be better suited to resist radial compressive forces in a stent once a stent is deployed. Thus, it would be advantageous, therefore, for the polymer of a stent to have a Tg that is above body temperature. However, such a polymer when it is below its Tg is susceptible to embrittlement and cracking during radial expansion.

As the temperature of a polymer is increased close to or above Tg, the energy barriers to rotation decrease and segmental mobility of polymer chains tends to increase. Consequently, polymers become more flexible, and thus, more resistant to embrittlement and cracking when they are at a temperature that is close to or above Tg. Therefore, it may be more desirable for a polymeric stent to be close to or above the Tg of the polymer when a stent is expanded.

However, polymers tend to be more susceptible to creep when they are close to or above the Tg. Additionally, creep can also result in a polymeric stent under stress. In particular, creep allows inward radial forces to permanently deform a stent radially inward. Therefore, creep reduces the effectiveness of a stent in maintaining a desired vascular patency.

In general, it is desirable to have a delivery system that allows a stent to be at least: (1) flexible and resistant to cracking during expansion; and (2) rigid and resistant to compressive forces so as to maintain vascular patency after deployment at an implant site.

Various embodiments of a method and system for delivering a stent in a bodily lumen that meet these criteria are disclosed. In general, the criteria may be met in part by using a stent fabricated from a polymer that is rigid at body temperature, e.g., a polymer with a Tg greater than body temperature. The other criterion may be met by increasing the flexibility of the stent during expansion such that formation of cracks in the stent upon its expansion is reduced or eliminated. This may be accomplished by heating a stent to a temperature close to, at, or above its Tg.

The invention provides for heating a stent before, during and/or after deployment to dramatically improve the mechanical stability of a deployed polymeric stent. By expanding the stent at an elevated temperature, the deployed stent exhibits less cracking, better mechanical properties, and more uniformity to the stent structure. Traditional techniques for deploying a stent at an elevated temperature require the use of heated contrast media, which is cumbersome and difficult in practice, so the technique has failed to gain acceptance as a potential technique.

The invention reduces cracks in polymeric stents during deployment by providing a catheter that works to heat the stent before, during, and/or after delivery. In certain embodiments, an expandable member, or balloon for expanding a stent includes a light or more generally a radiation absorbing material. A light or radiation emitting elements is disposed in, on, or adjacent to the light absorbing material. The material absorbs light or radiation and heats up an attached stent mounted on the expandable member. In some embodiments, the stent contains light absorbing materials, and is heated by the light source directly. In further embodiments, both the expandable member and the stent contain light absorbing materials, thus allowing the stent to be heated by both the expandable member and the light.

In some embodiments, the catheter contains a light guide that guides or transports infrared light or light of another appropriate wavelength such as UV light, from a light source. The light guide can be disposed within the catheter so that light is transported, thereby heating the member which ultimately heats an attached stent.

In some embodiments, the stent has infrared or UV absorbing materials either coated on the stent or incorporated with the polymer of the stent. The infrared or UV light is absorbed by the stent, resulting in an increase in stent temperature.

Further embodiments may include an expandable member that is able to absorb infrared or UV light as well as a stent that can absorb infrared or UV light.

In some embodiments, the invention takes advantage of a catheter including a light guide to heat a stent before, during, or after deployment. The balloon catheter according to some embodiments of the invention contains a light guide that may be connected to a light source. In several embodiments, the light source is in an inflation mechanism that is used to inflate the balloon. Light, or radiation from a light source is transported to a light emitting element on the catheter, and to the balloon. The light may also be emitted onto a stent coated with light absorbent materials, and thereby heating the stent, before, during, or after stent deployment.

The light absorbing material for use in fabricating the balloon may be any material capable of absorbing the infrared energy irradiating from the light emitting element. In particular, the material is capable of absorbing energy that is sufficient to heat the stent prior to or during, or after stent deployment. In one embodiment, the balloon surface is fabricated to facilitate the localization of the heating effect by selectively having the absorbing materials on certain portions of the balloon to heat selected portions of the stent.

In certain embodiments, when it is desired to heat the stent directly with light from the light guide, the balloon has some portions that are transparent to the light, thereby allowing the light to reach the stent.

In several embodiments, the polymer stent contains or is coated with an infrared or UV absorbent material to facilitate the application of heat to selected portions of the stent.

In some embodiments, the stent is heated with a light source before or during deployment with a balloon catheter into the bodily lumen. The stent can then be held open under pressure for a certain time period while a light source is still in place, continuing to heat the stent.

As used herein, a "light guide" is a material that is able to transport, transmit, or guide light or the flow of light through the material. The light may originate at one location, for example internally or externally of a target subject, such as a patient, and be transmitted by the light guide in the patient to a site of action, for example, a balloon catheter. Generally, a light guide has a proximal end optically coupled to a source of light radiation and a distal end with a light radiating/emitting end surface. The term "light emitting end surface" may be interchanged with the term "light emitting element."

In some embodiments, the light guide can include an optical fiber. An optical fiber can be a filament made from a dielectric material such as glass or plastic. The light guide should be able to transmit infrared and/or UV light. In some embodiments, the light guide comprises quartz glass.

In several embodiments, the light guide may include one or more fibers. In one set of embodiments, the light guide is substantially flexible or is otherwise able to return to its original shape after being distorted in some fashion.

In some embodiments, the catheter may be formed along its entire length of a suitable transparent material, for example a suitable fluorocarbon, polyurethane, silicone, nylon, polyethelene, polyester, or other suitable transmitting materials, or only a selected length along the distal end of the catheter may be formed of such material.

Infrared energy travels at the speed of light without heating the air it passes through, (the amount of infrared radiation absorbed by carbon dioxide, water vapor and other particles in the air typically is negligible) and gets absorbed or reflected by objects it strikes. The temperature of the object as well as its physical properties will dictate the radiant efficiency and wavelengths emitted. The wavelength of infrared radiation is measured at about 0.70 µm and extends to about 1000 µm, although an advantageous range of wavelengths for infrared heating applications occurs between about 0.70 µm to about 10 µm.

Infrared heating is the transfer of thermal energy in the form of electromagnetic waves. An object can absorb the radiation at some wavelength, reflect radiation at other wavelengths, and re-radiate at other wavelengths. The absorbed radiation which is converted to thermal energy heats the object. One range of wavelengths for infrared heating applications falls within the range of about 0.7 to about 10 µm on the electromagnetic spectrum and include short-wave, medium-wave or long-wave. The medium to long range wavelengths are advantageous since almost all materials to be heated or dried provide maximum absorption in the 3 to 10 µm region. Energy from an infrared heat source that also emits short-wave energy will typically emit 80% of its energy around the 1 µm wavelength. The penetration of infrared energy is a function of its wavelength. The shorter the wavelength, the greater its penetrating power.

In several embodiments the infrared light source is from a tungsten-halogen lamp. This configuration delivers visible light and infrared radiation, and may be used with a light guide comprising quartz glass.

In some embodiments, a coating used to absorb infrared energy may be a light absorbing metal layer disposed on the catheter balloon and/or on the stent. This metal layer is capable of absorbing selected wavelengths of light. For example, the metal layer can be an infrared absorbing metal layer or a visible light absorbing metal layer. The metal layer can be selected from metals already approved for use in implantable devices, such as, for example, gold or titanium. In one embodiment, the coating includes nano-sized gold bound to silica because this combination can readily absorb infrared light. The metal layer can then absorb the radiation from the light guide and convert that radiation into heat.

In some embodiments, the irradiating absorbing material may be mixed into the stent polymer to allow the absorption of infrared or UV light by the stent, thereby causing the stent to heat up.

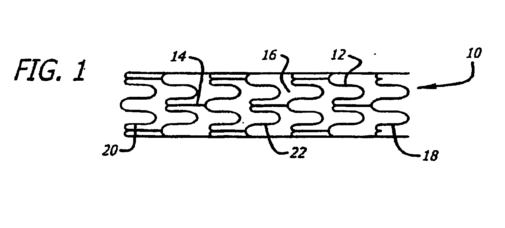

Referring to FIG. 1, a stent 10 is illustrated. Stent 10 can include a plurality of struts 12 connected by linking struts 14, with interstitial spaces 16 located in between the struts. The plurality of struts 12 can be configured in an annular fashion in discrete "rows" such that they form a series of "rings" throughout the body of stent 10. Thus, stent 10 can include a proximal ring 18, a distal ring 20 and at least one central ring 22.

As indicated above, stent 10 may be made from and/or coated with a biostable polymer or a bioerodable, biodegradable, bioadsorbable polymer or any combination thereof. In some embodiments, a polymeric stent can include other materials, such as layers or deposits of metallic material which can be bioerodable. The pattern should not be limited to what has been illustrated as other stent patterns are easily applicable with embodiments of the present invention.

In general, a stent pattern is designed so that the stent can be radially compressed (crimped) and radially expanded (to allow deployment). The stresses involved during compression and expansion are generally distributed throughout various structural elements of the stent pattern. As a stent expands, various portions of the stent can deform to accomplish a radial expansion.

Additionally, fabrication of an implantable medical device, such as a stent, may include forming a pattern that includes a plurality of interconnecting structural elements or struts on a tube. Polymer tubes may be formed by various types of methods, including, but not limited to extrusion or injection molding. In some embodiments, the diameter of the polymer tube prior to fabrication of an implantable medical device may be between about 0.2 mm and about 5.0 mm, or more narrowly between about 1 mm and about 3 mm-In some embodiments, forming a pattern on a tube may include laser cutting a pattern on the tube. Representative examples of lasers that may be used include, but are not limited to, excimer, carbon dioxide, and YAG. In other embodiments, chemical etching may be used to form a pattern on a tube.

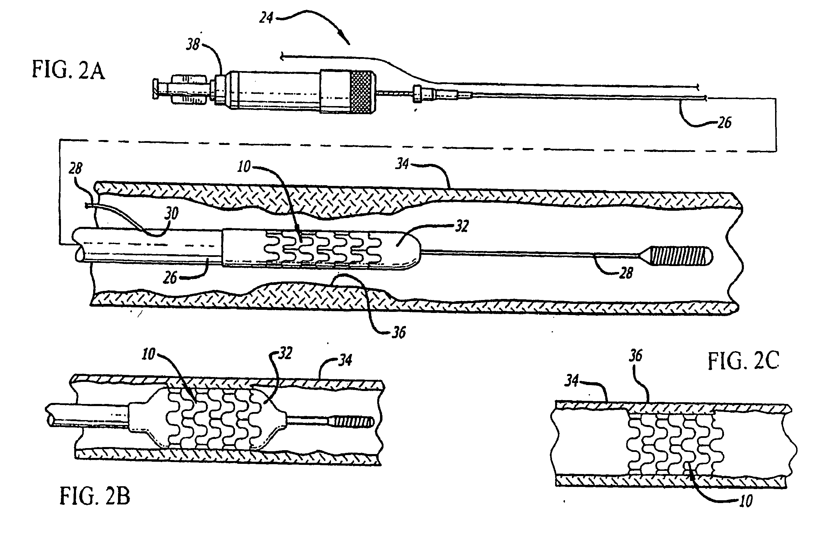

FIGS. 2A-2C illustrate an exemplary delivery system for delivering a balloon expandable stent into a tubular organ 34 according to several embodiments of the invention. FIG. 2A illustrates a catheter 24 which includes a proximal end 38 and a tip 26 including a light guide (not shown) on which an expandable member 32, e.g., a balloon, can be mounted on expandable member 32. An opening 30 is located on tip 26 through which a guidewire 28 is inserted to aid the user in the delivery of stent 10 which is mounted on expandable member 32.

In one deployment application, tip 26 of catheter 24, with a stent-balloon assembly mounted thereon, can be inserted into tubular organ 34 to treat a targeted region 36 of tubular organ 34. Once the stent-balloon assembly is positioned at targeted region 36, the expandable member 32 is heated using the light guide, thereby heating the stent 10. The stent-balloon assembly can be expanded by conveying a fluid into expandable member 32 from a fluid source (not shown in this figure) (see FIG. 2B). The fluid source can be an inflation device capable of conveying fluid into the catheter by creating a pressure gradient between the inflation device and the catheter. Subsequent to the positioning and expansion of stent 10, expandable member 32 can be deflated and withdrawn from targeted region 36 (see FIG. 2C) leaving stent 10 at targeted region 36.

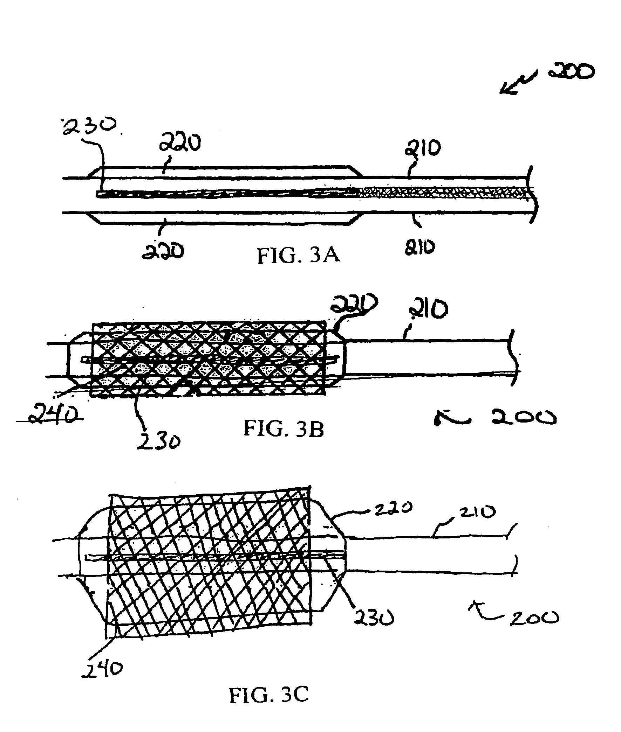

FIG. 3A depicts a side view of a balloon-catheter assembly 200 according to an embodiment of the invention. Balloon-catheter assembly 200 includes a support element or a delivery balloon 220 attached to a catheter 210. An infrared light irradiating element 230 is disposed within the catheter 210.

The stent mounted on the balloon-catheter assembly is then guided into place at a treatment site and heated by the light element in the catheter. FIG. 3B depicts a side view of balloon-catheter assembly 200 having infrared light irradiating element 230 and a stent 240 crimped onto balloon-catheter assembly 200 according to one embodiment of the invention. Once the balloon-catheter assembly 200 is positioned in the treatment site of the lumen, infrared light irradiating element 230 irradiates balloon 220 causing the balloon to heat up, thereby heating attached stent 240. In several embodiments, stent 240 having the ability to absorb the infrared light is directly heated by exposure of stent 240 to infrared light which is delivered through balloon 220 by use of a light guide with a fiber optic tip 230. Stent 240 is sufficiently heated for a specified time to a temperature that depends on the polymer in the stent. In one embodiment, the infrared light irradiating element 230, that may be connected to, or be the distal end of a light guide that extends through the catheter from a light source, increases the temperature of stent 240, either directly wherein the stent absorbs the light, or indirectly by heating balloon 220 to which the stent is attached, to a temperature between body temperature, 37°C, to a temperature below the glass transition temperature of the polymer. In these embodiments, fewer cracks in the stent result from deployment because the stent is sufficiently heated above body temperature to reduce cracking. In another embodiment, the stent is heated to a temperature above the glass transition temperature of the stent. The infrared energy delivered to element 230 may be controlled to adjust the temperature to a desired or optimum temperature for stent deployment. In an embodiment, the optimum temperature is the temperature at which the least number of cracks are produced.

FIG. 3C depicts a side view of balloon 220 in the expanded state, pushing stent 240 into its expanded state.

In some embodiments, after heating and inflating the stent, the heating continues to be applied to the balloon and or directly to the stent for a period of time, thereby deploying a stent that exhibits relatively fewer cracks.

Infrared energy is delivered through element 230, thereby heating balloon 220. Heated balloon 220 then heats stent 240. In one embodiment, the stent can be heated prior to expanding the stent. Alternatively, the heating can be started during expansion of the stent. In another embodiment, the stent can be heated prior to expansion and heating can continue during and optionally after expansion. In such embodiments, the stent can be heated to the target temperature prior to expansion. Alternatively, the stent can be heated to a temperature below the target temperature prior to expansion and heated to the target temperature during or after expansion.

In some embodiments, the stent can be heated to a target temperature at a relatively constant rate. Alternatively, the heating rate of the stent can be nonlinear, e.g., slow initially and fast subsequently. In a further embodiment, the heating can be performed in states or step-wise with equilibrium periods between increases in temperature.

Additionally, the temperature of the balloon or stent can be monitored during the heating process. In one embodiment, thermocouple leads (not shown) can be coupled within or on the balloon to monitor the temperature. The leads can be in communication through wires extending with the catheter with a system that displays the temperature.

In some embodiments, the heating can be manually controlled by an operator. In other embodiments, the heating can be preprogrammed into a control system that automatically adjusts the heating rate. In some embodiments, the heating rate and/or temperature of the stent can be controlled by a control system. The power source and temperature sensors can be in communication with the control system. Based on input parameters, such as measured temperature, the heating can be controlled to obtain desired heating of the stent. The control system can employ a feedback control mechanism known to one of ordinary skill in the art.

During or after the stent is heated, the balloon is deflated and removed, deploying a radially expanded stent that exhibits relatively fewer cracks.

The present invention can be generally applied to, but not limited to, self-expandable stents, balloon-expandable stents, stent-grafts, vascular grafts, and generally tubular implantable medical devices. A stent specifically designed and intended solely for localized delivery of a therapeutic agent is also within the scope of this invention. Thus, the stent may also be used to open a lumen within an organ in a mammal, maintain lumen patency, and/or reduce the likelihood of the narrowing of a lumen.

In one embodiment, the polymer for use in forming the stent scaffolding and/or the stent coating may be configured to degrade after implantation by fabricating the stent either partially or completely from biodegradable polymers. A biodegradable stent may remain in the body until its intended function of, for example, maintaining vascular patency and/or drug delivery is accomplished. For biodegradable polymers used in coating applications, after completion of the process of degradation, erosion, and absorption, no polymer remains on the stent. In some embodiments, very negligible traces of residue are left behind. In one embodiment, poly(L-lactide) is used to fabricate the stent.

It is understood that after the process of degradation, erosion, absorption, and/or resorption has been completed, no part of the stent will remain or in the case of coating applications on a biostable scaffolding, no polymer will remain on the device. In some embodiments, very negligible traces or residue may be left behind. For stents made from a biodegradable polymer, the stent is intended to remain in the body for a duration of time until its intended function of, for example, maintaining vascular patency and/or drug delivery is accomplished.

The underlying structure or substrate of an implantable medical device, such as a stent can be completely or at least in part made from a biodegradable polymer or combination of biodegradable polymers, a biostable polymer or combination of biostable polymers, or a combination of biodegradable and biostable polymers. Additionally, a polymer-based coating for a surface of a device can be a biodegradable polymer or combination of biodegradable polymers, a biostable polymer or combination of biostable polymers, or a combination of biodegradable and biostable polymers.

Representative examples of polymers that may be used to fabricate or coat an implantable medical device include, but are not limited to, poly(N-acetylglucosamine) (Chitin), Chitosan, poly(hydroxyvalerate), poly(lactide-co-glycolide), poly(hydroxybutyrate), poly(hydroxybutyrate-co-valerate), polyorthoester, polyanhydride, poly(glycolic acid), poly(glycolide), poly(L-lactic acid), poly(L-lactide), poly(D,L-lactic acid), poly(D,L-lactide), poly(caprolactone), poly(trimethylene carbonate), polyester amide, poly(glycolic acid-co-trimethylene carbonate), co-poly(ether-esters) (e.g. PEO/PLA), polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates, polyoxymethylenes, polyimides, polyethers, polyurethanes, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose. Another type of polymer based on poly(lactic acid) that can be used includes graft copolymers, and block copolymers, such as AB block-copolymers ("diblock-copolymers") or ABA block-copolymers ("triblock-copolymers"), or mixtures thereof.

Additional representative examples of polymers that may be especially well suited for use in fabricating or coating an implantable medical device include ethylene vinyl alcohol copolymer (commonly known by the generic name EVOH or by the trade name EVAL), poly(butyl methacrylate), poly(vinylidene fluoride-co-hexafluororpropene) (e.g., SOLEF 21508, available from Solvay Solexis PVDF, Thorofare, NJ), polyvinylidene fluoride (otherwise known as KYNAR, available from ATOFINA Chemicals, Philadelphia, PA), ethylene-vinyl acetate copolymers, and polyethylene glycol.

Implantable medical devices such as stents are typically subjected to stress during use, both before and during treatment. "Use" includes, but is not limited to, manufacturing, assembling (e.g., crimping a stent on a catheter), delivery of a stent into and through a bodily lumen to a treatment site, and deployment of a stent at a treatment site. Both a scaffolding and a coating on a scaffolding experience stress that result in strain in the scaffolding and/or coating. For example, during deployment, the scaffolding of a stent can be exposed to stress caused by the radial expansion of the stent body. In addition, the scaffolding and/or coating may be exposed to stress when it is mounted on a catheter from crimping or compression of the stent.

In some embodiments, the bending portions of the stent are selectively heated because they are subjected to the most stress. In several embodiments this is accomplished by selectively coating the balloon with infrared absorbing material such that only select portions of an attached stent are heated. Further embodiments include selectively coating the stent with infrared materials so that only selected portions of the stent are directly heated by exposure to infrared light.

Implantable medical devices, such as stents, that relate to the embodiments described herein typically include an underlying scaffolding or substrate. The underlying structure or substrate of the device can be of virtually any design. The substrate may have a polymer-based coating that may contain, for example, an active agent or drug for local administration at a diseased site. The active agent can be any substance capable of exerting a therapeutic or prophylactic effect.

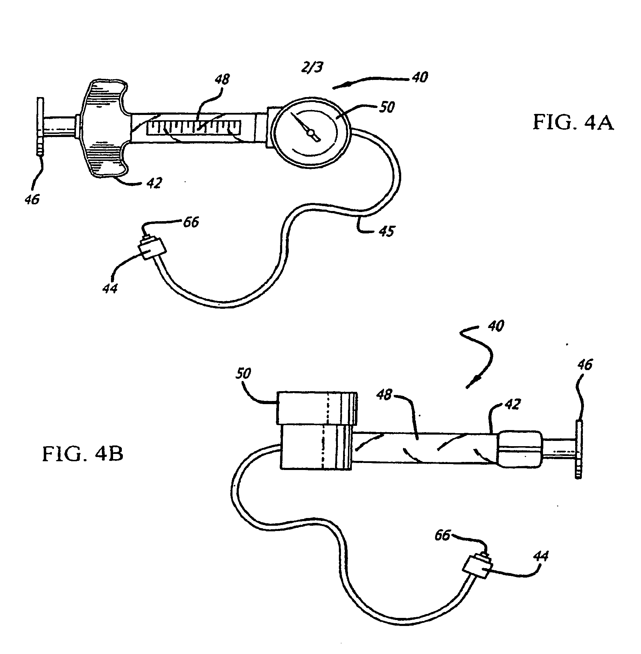

FIGS. 4A-4B illustrate an exemplary inflation device 40 which can be used to expand a stent-balloon assembly. Inflation device 40 can convey fluid into catheter 24 and expand expandable member 32 by creating a pressure gradient between inflation device 40 and catheter 24. Inflation device 40 can include a pressure injector at a first end 42 and an exit port 66 at the tip of a flexible tube 45. Inflation device 40 further includes a chamber 48 and a pressure gauge 50. A light source (not shown) may be coupled to, within, or on the inflation device. In some embodiments, a light guide connected to a catheter is coupled to inflation device 40. Chamber 48 can be filled with a fluid for use in expanding an expandable member 32. Typically, the fluid is a liquid, however, a gas may also be used. Chamber 48 can have a capacity, for example, between about 10 ml and 40 ml.

In addition to expanding a balloon, the fluid may assist a user in visualizing the catheter and balloon during delivery. As indicated above, a fluid that is visible to an imaging technique, such as x-ray fluoroscopy or magnetic resonance imaging (MRI), may be used to inflate a balloon. Such fluids are referred to as contrast agents. Thus, a contrast agent can include a radiopaque agent or a magnetic resonance imaging agent. "Radiopaque" refers to the ability of a substance to absorb x-rays. An MRI agent has a magnetic susceptibility that allows it to be visible with MRI.

In some applications, exit port 66 of inflation device 40 can be coupled to a catheter for inflation (and deflation) of a balloon during deployment of a stent. A fluid for inflating expandable member 32 can be disposed in chamber 48. During a delivery procedure, the pressure injector 46 can be depressed for injecting fluid 68 into catheter 24 and expandable member 32 for inflation thereof. Pressure gauge 50 gives a user an indication of the pressure so that it can be monitored to prevent balloon burst.

Inflation device 40 can be made of a durable plastic such as polycarbonate or metal such as stainless steel. Moreover, inflation device 40 may be reusable or disposable. Device 40 is typically disposable after a one single use due to the highly critical nature of the catherization procedure. Generally, inflation device 40 can be between 10.16 cm (four inches) to 25.4 cm (ten inches) in length.

In some embodiments, balloon expansion may be performed in stages or measured intervals. Such a procedure tends to reduce or prevent vessel injury. Additionally, performing the expansion in stages may allow a stent to equilibrate at higher temperatures as it is heated.

While particular embodiments of the present invention have been shown and described, it will be obvious to those skilled in the art that changes and modifications can be made without departing from this invention in its broader aspects. Therefore, the appended claims are to encompass within their scope all such changes and modifications as fall within the scope of this invention.

## Claims

1. A delivery assembly comprising:
a catheter (24) comprising an expandable member (32) disposed on the catheter, wherein the expandable member is capable of absorbing infrared energy and generating heat;
an infrared energy-irradiating element disposed within the catheter (24) for transmitting infrared energy to the expandable member (32), wherein heat generated from the infrared energy is configured to heat a stent (10) when the stent is mounted on the expandable member (32), thereby heating the stent to a temperature above body temperature, wherein the expendable member comprises a coating which absorbs the infrared energy, and wherein the coating is a metal layer.

2. The delivery assembly of claim 1, wherein the expandable member comprises a delivery balloon.

3. The delivery assembly of claim 1, wherein the heat increases the temperature of the stent close to, at, or above a glass transition temperature of the polymer of the stent.

4. The delivery assembly of claim 1, wherein the expandable member comprises a fluid, and wherein the fluid expands the expandable member.

5. The delivery assembly of claim 1, wherein the infrared energy-irradiating element is coupled to or comprises a distal end of a light guide disposed within the catheter.

6. The delivery assembly of claim 5, wherein the light guide comprises a proximal end optically coupled to an infrared energy-irradiating source and a distal end with the infrared energy-irradiating element.

7. The delivery assembly of claim 6, wherein the infrared energy-irradiating source is a tungsten-halogen lamp.

8. The delivery assembly of claim 1, wherein the infrared energy-irradiating element further comprises a fiber optic cable.

9. The delivery assembly of claim 1, wherein the metal layer comprises gold or titanium.

10. The delivery assembly of claim 9, wherein portions of the expandable member are selectively coated to allow selective heating of those portions.

11. The delivery assembly of claim 1, further comprising a polymeric stent disposed over the expandable member, wherein the stent comprises a biostable and/or bioabsorbable polymer.

12. The delivery assembly of claim 11, wherein the stent comprises a material capable of absorbing the infrared energy from the infrared energy-irradiating element.

13. The delivery assembly of claim 12, wherein the infrared energy absorbing material comprises gold or titanium.

14. The delivery assembly of claim 11, wherein portions of the stent are selectively coated with the infrared absorbing material to allow selective heating of those portions.

15. The delivery assembly of claim 14, wherein the coated portions are the bends of the stent.

## Patentansprüche

1. Abgabevorrichtung mit:
einem Katheter (24), der ein ausdehnbares auf dem Katheter angebrachtes Teil (32) besitzt, wobei das ausdehnbare Teil Infrarotenergie absorbieren und Wärme erzeugen kann;
einem Infrarotenergie ausstrahlenden Bauelement, das im Katheter (24) zur Übertragung von Infrarotenergie zum ausdehnbaren Teil (32) angebracht ist, wobei die von der Infrarotenergie erzeugte Wärme dafür konzipiert ist, einen Stent (10) zu wärmen, wenn der Stent auf dem ausdehnbaren Teil (32) aufgezogen ist, wodurch der Stent auf eine Temperatur oberhalb der Körpertemperatur erwärmt wird, bei dem das ausdehnbare Teil eine Beschichtung besitzt, die die Infrarotenergie absorbiert, und bei dem die Beschichtung eine Metallschicht ist.

2. Abgabevorrichtung nach Anspruch 1, bei der das ausdehnbare Teil einen Abgabeballon umfasst.

3. Abgabevorrichtung nach Anspruch 1, bei der die Wärme die Stenttemperatur nahezu auf, auf oder über eine Glasübergangstemperatur des Stentpolymers erhöht.

4. Abgabevorrichtung nach Anspruch 1, bei der das ausdehnbare Teil ein Fluid enthält, und bei der das Fluid das ausdehnbare Teil ausweitet.

5. Abgabevorrichtung nach Anspruch 1, bei der das Infrarotenergie ausstrahlende Bauelement mit einem distalen Ende eines im Katheter vorhandenen Lichtleiters gekoppelt ist oder dieses enthält.

6. Abgabevorrichtung nach Anspruch 5, bei der der Lichtleiter ein proximales Ende, das optisch an eine Infrarotenergie ausstrahlende Quelle gekoppelt ist, und ein distales Ende mit dem Infrarotenergie ausstrahlenden Bauelement besitzt.

7. Abgabevorrichtung nach Anspruch 6, bei der die Infrarotenergie ausstrahlende Quelle eine Wolfram-Halogen-Lampe ist.

8. Abgabevorrichtung nach Anspruch 1, bei der das Infrarotenergie ausstrahlende Bauelement weiterhin ein Glasfaserkabel umfasst.

9. Abgabevorrichtung nach Anspruch 1, bei der die Metallschicht Gold oder Titan enthält.

10. Abgabevorrichtung nach Anspruch 9, bei der Stellen des ausdehnbaren Teils selektiv beschichtet sind, um das selektive Erwärmen dieser Stellen zu ermöglichen.

11. Abgabevorrichtung nach Anspruch 1, die weiterhin einen über das ausdehnbare Teil gezogenen Polymerstent enthält, bei der der Stent ein biostabiles und/oder bioresorbierbares Polymer enthält.

12. Abgabevorrichtung nach Anspruch 11, bei dem der Stent ein Material enthält, das die Infrarotenergie vom Infrarotenergie ausstrahlenden Bauelement absorbieren kann.

13. Abgabevorrichtung nach Anspruch 12, bei der das Infrarotenergie absorbierende Material Gold oder Titan enthält.

14. Abgabevorrichtung nach Anspruch 11, bei der Stellen des Stent selektiv mit dem Infrarot absorbierendem Material beschichtet sind, um das selektive Erwärmen dieser Stellen zu ermöglichen.

15. Abgabevorrichtung nach Anspruch 14, bei der die beschichteten Stellen die Krümmungen des Stent sind.

## Revendications

1. Ensemble d'apport comprenant :
un cathéter (24) comprenant un membre expansible (32) disposé sur le cathéter, dans lequel le membre expansible est susceptible d'absorber l'énergie infrarouge et de générer de la chaleur ;
un élément irradiant de l'énergie infrarouge disposé au sein du cathéter (24) pour transmettre l'énergie infrarouge au membre expansible (32) dans lequel la chaleur générée à partir de l'énergie infrarouge est conçue pour chauffer un stent (10) lorsque le stent est monté sur le membre expansible (32) en chauffant de ce fait le stent à une température au-dessus de la température du corps, dans lequel le membre expansible comprend un revêtement qui absorbe l'énergie infrarouge, et dans lequel le revêtement est une couche de métal.

2. Ensemble d'apport selon la revendication 1, dans lequel le membre expansible comprend un ballonnet d'apport.

3. Ensemble d'apport selon la revendication 1, dans lequel la chaleur augmente la température du stent près de, au niveau de, ou au-dessus d'une température de transition vitreuse du polymère du stent.

4. Ensemble d'apport selon la revendication 1, dans lequel le membre expansible comprend un fluide, et dans lequel le fluide dilate le membre expansible.

5. Ensemble d'apport selon la revendication 1, dans lequel l'élément irradiant de l'énergie infrarouge est couplé à ou comprend une extrémité distale d'un guide lumineux disposé au sein du cathéter.

6. Ensemble d'apport selon la revendication 5, dans lequel le guide lumineux comprend une extrémité proximale optiquement couplée à une source irradiant de l'énergie infrarouge et une extrémité distale avec l'élément irradiant de l'énergie infrarouge.

7. Ensemble d'apport selon la revendication 6, dans lequel la source irradiant de l'énergie infrarouge est une lampe tungstène-halogène.

8. Ensemble d'apport selon la revendication 1, dans lequel l'élément irradiant de l'énergie infrarouge comprend en outre un câble à fibre optique.

9. Ensemble d'apport selon la revendication 1, dans lequel la couche de métal comprend de l'or ou du titane.

10. Ensemble d'apport selon la revendication 9, dans lequel des parties du membre expansible sont revêtues de façon sélective pour permettre un chauffage sélectif de ces parties.

11. Ensemble d'apport selon la revendication 1, comprenant en outre un stent polymère disposé sur le membre expansible, dans lequel le stent comprend un polymère biostable et/ou bioabsorbable.

12. Ensemble d'apport selon la revendication 11, dans lequel le stent comprend un matériau susceptible d'absorber l'énergie infrarouge provenant de l'élément irradiant de l'énergie infrarouge.

13. Ensemble d'apport selon la revendication 12, dans lequel le matériau absorbant l'énergie infrarouge comprend de l'or ou du titane.

14. Ensemble d'apport selon la revendication 11, dans lequel des parties du stent sont revêtues de façon sélective avec le matériau absorbant l'infrarouge pour permettre un chauffage sélectif de ces parties.

15. Ensemble d'apport selon la revendication 14, dans lequel les parties revêtues sont les plis du stent.
